# EUROPÄISCHE PATENTSCHRIFT

(11) **EP 3 420 882 B1**
(45) Veröffentlichungstag und Bekanntmachung des Hinweises auf die Patenterteilung: **30.12.2020**
(21) Anmeldenummer: 18176484.6
(22) Anmeldetag: 07.06.2018
(51) Int. Cl.: A61B 1/00, A61B 1/002, A61B 1/04, G02B 13/00, G02B 23/24

(54) **KAMERAOBJEKTIV FÜR EIN ENDOSKOP UND ENDOSKOP**
ENDOSCOPE AND LENS FOR A CAMERA FOR ENDOSCOPE
OBJECTIF DE CAMÉRA POUR UN ENDOSCOPE ET ENDOSCOPE

(30) Priorität: 16.06.2017 DE 102017113274
(43) Veröffentlichungstag der Anmeldung: 02.01.2019
(73) Patentinhaber: avateramedical GmbH, 07745 Jena (DE)
(72) Erfinder: KHETTAL, Ali, 12435 Berlin (DE); WEISE, Fabian, 10437 Berlin (DE)
(74) Vertreter: Schaumburg und Partner Patentanwälte mbB

(56) Entgegenhaltungen:
- EP-A1- 2 293 130
- EP-A1- 3 162 273
- US-A1- 2016 004 054

## Beschreibung

Die Erfindung betrifft ein Kameraobjektiv für ein Endoskop mit einem objektseitigen ersten Prisma, einem bildseitigen zweiten Prisma, einem objektseitig des ersten Prismas angeordneten ersten Linsensystem, einem bildseitig des ersten Prismas angeordneten zweiten Linsensystem und einer am bildseitigen Ende des Kameraobjektivs parallel zur Längsachse eines Endoskopschafts des Endoskops angeordneten Sensoroberfläche.

Endoskope werden insbesondere in der minimalinvasiven Chirurgie verwendet, um dem operierenden Arzt Einblick in die Körperregion zu gewähren, in der das Operationsgebiet liegt. Verwendet werden sowohl monokulare Endoskope als auch stereoskopische Endoskope, welche über zwei optische Kanäle einen räumlichen Eindruck der Tiefe liefern, der mit monokularen Endoskopen nicht möglich ist.

Am distalen Ende eines Endoskopschafts ist typischerweise ein Objektiv angeordnet, welches das vom zu beobachtenden Objekt ausgehende Licht sammelt und ein reelles Zwischenbild des Objektes erzeugt. Dieses Zwischenbild wird mit Hilfe eines dem Objektiv nachgeordneten optischen Relaissystems zum proximalen Ende des Endoskopschafts übertragen. Am proximalen Ende des Endoskopschafts ist ein Okular angeordnet, welches das reelle Zwischenbild für das menschliche Auge abbildet. Anstelle eines rein optischen Okulars kann das Okular auch einen Kameraadapter mit einem geeigneten Bildsensor endhalten, der die Bildbetrachtung auf einem Bildschirm und die Speicherung von Videoinformationen ermöglicht.

Typischerweise werden sowohl monokulare Endoskope als auch stereoskopische Endoskope, die zusätzliche Tiefeninformationen liefern, verwendet, um dadurch ein genaues Operieren zu ermöglichen.

Aus dem Dokument JP 6-160731 ist ein stereoskopisches Endoskop bekannt, welches zwei gleichartige Relaisoptiken aufweist, die ein Bild optisch von einem Objektiv zu einem Okular leiten. Das Okular enthält ein Prismen-System, welches mit Hilfe zweier Strahlumleitungen die von der Relaisoptik erzeugten reellen Zwischenbilder auf zwei Sensoroberflächen, die orthogonal zur optischen Achse O des Endoskops angeordnet sind, parallel versetzt abbildet.

Eine ähnliche Anordnung mit zwei verstellbaren Prismen zur Strahlumleitung auf orthogonal zur optischen Achse angeordnete Sensoroberflächen ist in dem Dokument EP 0 667 547 A2 offenbart.

Im Stand der Technik wird zudem ein Okular in Kombination mit einem Adapter für ein Endoskop offenbart, welches durch einmalige Strahlablenkung mit Hilfe eines um 45° geneigten Spiegels das Zwischenbild der Relaisoptik auf eine parallel zur optischen Achse angeordnete Sensoroberfläche abbildet.

In dem Dokument DE 35 29 026 C2 ist ein Kameraobjektiv für ein Endoskop offenbart, welches durch vierfache Strahlablenkung mit Hilfe eines Prismen-Systems das Zwischenbild der Relaisoptik auf eine parallel zur optischen Achse angeordnete Sensoroberfläche abbildet. In dem Dokument DE 35 29 026 C2 ist zudem ein Kameraobjektiv mit dreifacher Strahlumlenkung offenbart, bei dem zwischen der zweiten und der dritten Strahlumlenkung parallel zur optischen Achse optische Bauteile angeordnet sind.

Aus dem Dokument EP3162273 ist ein Kameraobjektiv entsprechend dem Oberbegriff von Anspruch 1 bekannt.

In der Endoskopie sollte der Schaft, an dessen proximalem Ende sich das Objektiv befindet und der die Relaisoptik umfasst, einen möglichst kleinen Durchmesser haben. Demgegenüber sollte die Sensoroberfläche technisch bedingt eine gewisse Größe aufweisen. Das Zwischenbild der Relaisoptik muss vor der Abbildung auf die Sensoroberfläche vergrößert werden. Diese Vergrößerung geht typischerweise mit einem Verlust an optischer Qualität einher.

Es ist Aufgabe der Erfindung, ein Kameraobjektiv für ein Endoskop anzugeben, das einen kompakten Aufbau hat und mit dessen Hilfe ein Verlust an optischer Qualität bei der Vergrößerung vermieden werden kann.

Diese Aufgabe wird durch ein Kameraobjektiv mit den Merkmalen des Anspruchs 1 und ein monokulares Endoskop oder stereoskopisches Endoskop mit den Merkmalen des Anspruchs 15 gelöst. Vorteilhafte Weiterbildungen sind in den abhängigen Ansprüchen angegeben.

Das erfindungsgemäße Kameraobjektiv umfasst:
- eine erste optische Achse und eine zweite optische Achse, die relativ zur ersten optischen Achse versetzt ist,
- ein objektseitiges erstes Prisma und ein bildseitiges zweites Prisma,
- ein objektseitig des ersten Prismas angeordnetes erstes Linsensystem und ein bildseitig des ersten Prismas angeordnetes zweites Linsensystem, wobei das erste Linsensystem längs der ersten optischen Achse angeordnet ist, und wobei das zweite Linsensystem längs der zweiten optischen Achse angeordnet ist, und
- eine am bildseitigen Ende des Kameraobjektivs parallel zur ersten optischen Achse angeordneten Sensoroberfläche, wobei das erste Linsensystem eine Okularoptik und das zweite Linsensystem eine Adapteroptik bildet,
- wobei das erste Prisma und das zweite Prisma dazu ausgebildet sind, eine erste bis dritte Strahlumlenkung als eine dreifache Strahlumlenkung zu bewirken, so dass mit Hilfe des ersten Prismas die erste und zweite Strahlumlenkung als eine zweifache Strahlumlenkung von der ersten optischen Achse auf die zweite optischen Achse und mit Hilfe des zweiten Prismas die dritte Strahlablenkung als eine einfache Strahlumlenkung von der zweiten optischen Achse auf die parallel zur ersten optischen Achse angeordnete Sensoroberfläche bewirkt wird,
- dadurch gekennzeichnet, dass das erste Linsensystem eine bikonkave erste Linse, eine bikonvexe
   zweite Linse, eine als Stablinse ausgebildete dritte Linse, eine plan-konkave vierte Linse und eine bikonvexe fünfte Linse in dieser Reihenfolge von der Objektseite her gesehen enthält,
- und dass das zweite Linsensystem eine konvex-plane sechste Linse, eine als Meniskuslinse ausgebildete siebente Linse, eine bikonvexe achte Linse und eine bikonkave neunte Linse in dieser Reihenfolge von der Objektseite her gesehen enthält.

Die dreifache Strahlumlenkung ermöglicht eine besonders kompakte Anordnung der optischen Elemente innerhalb des Kameraobjektivs. Durch die exzentrische Anordnung des zweiten Linsensystems (Adapteroptik) wird insbesondere in einem Kameraobjektiv für ein stereoskopisches Endoskop ein besonders kompakter Aufbau erreicht. Die Anordnung der Sensoroberfläche parallel zur optischen Achse des Endoskops erlaubt sowohl in monokularen als auch stereoskopischen Endoskopen einen kompakten Aufbau des distalen Endes. Das erste Linsensystem (Okularoptik) ist zudem so ausgeformt, dass sie eventuell vorhandene Bildfehler des Objektivs und/oder der Relaisoptik korrigiert. Insbesondere eine noch vorhandene positive Bildfeldwölbung wird durch die negative Bildfeldkrümmung der Okularoptik ausgeglichen. Hierdurch wird ein kompakter Aufbau des Kameraobjektivs erreicht. Gleichzeitig kann mit dessen Hilfe ein Verlust an optischer Qualität bei der Vergrößerung vermieden werden. Insbesondere handelt es sich bei dem ersten Linsensystem um eine Okularoptik und bei dem zweiten Linsensystem um eine Adapteroptik. Die Okularoptik bildet zusammen mit der Adapteroptik das Kameraobjektiv.

Die Anordnung einer Stablinse als Teil der Okularoptik erlaubt außerdem eine Anpassung der Länge des Endoskops, ohne dass der Schaftdurchmesser vergrößert werden muss.

Die bikonkave erste Linse und die bikonvexe zweite Linse der Okularoptik sind in einer möglichen, vorteilhaften Ausgestaltung miteinander verkittet und chromatisch korrigiert. Ferner können die dritte Linse, die plan-konkave vierte Linse und die bikonvexe fünfte Linse der Okularoptik miteinander verkittet und chromatisch korrigiert sein.

Ferner ist es vorteilhaft, wenn die bikonvexe achte Linse und die bikonkave neunte Linse der Adapteroptik ebenfalls miteinander verkittet und chromatisch korrigiert sind.

In einer vorteilhaften Weiterbildung sind die Stablinse und die plan-konkave vierte Linse der Okularoptik miteinander verkittet. Diese Verkittung optischer Elemente zu einem Kittglied erlaubt eine einfache Montage derselbigen im Endoskop. Insbesondere die Ausgestaltung der Stablinse und des Kittglieds mit jeweils einer Planfläche erlaubt es, das Pointing in einem Justageschritt durch laterale Verschiebung einzustellen. Das Kittglied liegt dafür auf und benötigt keine weitere Fassung, da dies nach der Justage direkt verkittet werden kann. Dies ist insbesondere für Stereoendoskope wichtig. Durch die axiale Verschiebung der Okularoptik 4 kann die Schärfe insbesondere bei der Montage auf einen Abstand eingestellt werden.

Es ist vorteilhaft, parallel zur Sensoroberfläche eine planparallele Glasplatte zum Schutz derselbigen anzuordnen.

Beispielsweise weisen eine oder mehrere Linsen der Okularoptik und/oder eine oder mehrere Linsen der Adapteroptik auf ihren freiliegenden, luftliegenden Außenflächen eine Antireflexionsschicht auf. Dies dient der Minimierung von Streulicht und der damit verbundenen Reduzierung der optischen Qualität des Endoskops. Ferner erhöht die Antireflexionsschicht die optische Transmission.

Beispielsweise umfassen eine oder mehrere Oberflächen des ersten bzw. zweiten Prismas eine Hochreflexionsschicht. Dies dient dazu, möglichst viel Licht bei den Strahlumlenkungen zu reflektieren und die optische Qualität des Systems zu erhöhen.

Es ist vorteilhaft, wenn eine oder mehrere parallel zur ersten optischen Achse und senkrecht zur Sensoroberfläche stehende Oberflächen des ersten Prismas und/oder des zweiten Prismas eine matt schwarze Beschichtung aufweisen. Auch dies dient der Minimierung von Streulicht und der damit verbundenen Reduzierung der optischen Qualität des Endoskops.

Ferner ist vorteilhaft, wenn eine oder mehrere optische Komponenten des Kameraobjektivs aus einem Flintglas oder Kronglas bestehen. Die hohe Dispersion von Flintgläsern einerseits erlaubt die Konstruktion des jeweiligen Linsensystems mit gewünschten achromatischen Eigenschaften, insbesondere durch die Kombination leichter und schwerer Flintgläser mit unterschiedlicher Abbe- und Brechzahl. Andererseits ist die Verwendung von insbesondere Barium-Kronglas auf Grund seiner optischen Übertragungseigenschaften vorteilhaft.

In einer weiteren vorteilhaften Weiterbildung ist in der Adapteroptik eine Vorrichtung zur Justierung von Bildschärfe und Vergrößerung angeordnet. Insbesondere in einem stereoskopischen Endoskop können somit die Bildschärfe und Vergrößerung für jeden der beiden Bildkanäle separat eingestellt werden. Die Bildschärfe kann auch durch Verschieben des Bildsensors eingestellt werden.

Ferner betrifft die Erfindung ein stereoskopisches Kameraobjektiv, welches zwei paarweise angeordnete Kameraobjektive nach oben beschriebener Art enthält.

Ein weiterer Aspekt der Erfindung betrifft ein monokulares Endoskop oder stereoskopisches Endoskop. Das monokulare Endoskop umfasst ein im Vorhergehenden beschriebenes Kameraobjektiv. Das stereoskopische Endoskop umfasst das gerade genannte stereoskopische Kameraobjektiv.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Figur 1: ein Kameraobjektiv gemäß einem Ausführungsbeispiel;
- Figur 2: eine beispielhafte Okularoptik des Kameraobjektivs nach Figur 1;

- Figur 3: das Kameraobjektiv nach Figur 1 mit Darstellungsebene orthogonal zur Sensoroberfläche ohne Darstellung der Okularoptik;
- Figur 4: das Kameraobjektiv nach Figur 1 mit Darstellungsebene parallel zur Sensoroberfläche ohne Darstellung der Okularoptik;
- Figur 5: ein monokulares Endoskop gemäß einem Ausführungsbeispiel; und
- Figur 6: ein stereoskopisches Endoskop gemäß einem Ausführungsbeispiel.

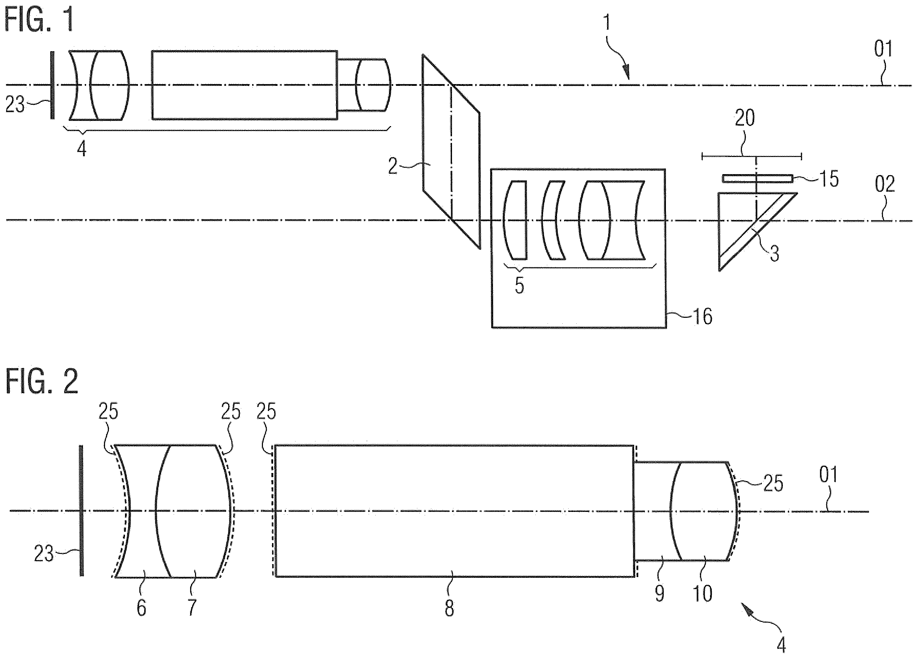

Ein Kameraobjektiv 1 ist in einer möglichen Ausgestaltung in Figur 1 schematisch dargestellt. Das Kameraobjektiv 1 umfasst eine Okularoptik 4, ein objektseitiges Prisma 2, eine Adapteroptik 5, ein bildseitiges Prisma 3 und eine Sensoroberfläche 20. Gezeigt ist auch das Zwischenbild 23 eines dem Kameraobjektiv 1 vorgeschalteten, in Figur 1 nicht gezeigten optischen Relaissystems.

Die Okularoptik 4 umfasst mehrere Linsen 6 bis 10, insbesondere eine Stablinse 8, und ist in Figur 2 näher beschrieben. Vorteilhafterweise ist die Okularoptik 4 derart ausgestaltet, Bildfehler einer vorgeschalteten Optik, beispielsweise des in Figur 1 nicht gezeigten optischen Relaissystems, zu korrigieren. Bei diesen Bildfehlern kann es sich beispielsweise um eine Bildfeldwölbung handeln.

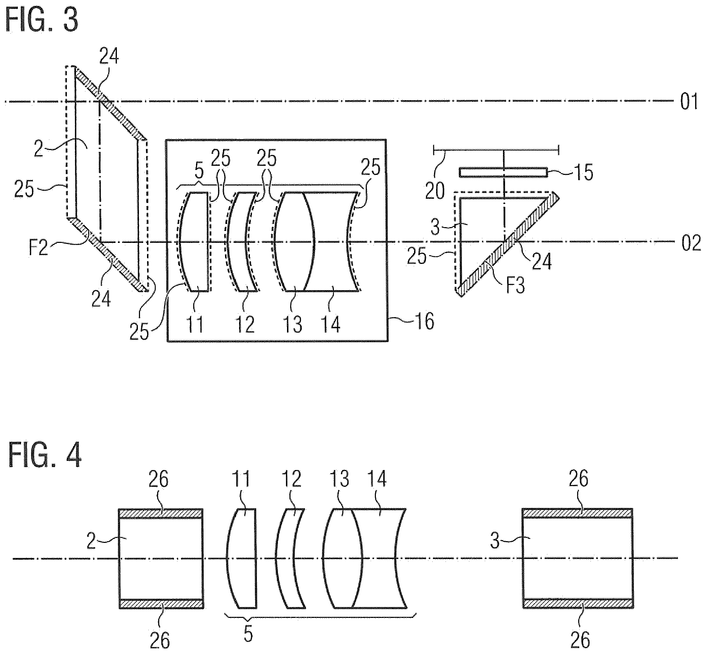

Ferner enthält das Kameraobjektiv 1 eine parallel bzw. exzentrisch zur optischen Achse O1 des Endoskopschafts des Endoskops angeordnete Adapteroptik 5. Die Adapteroptik 5 umfasst mehrere Linsen 11 bis 14. Zum Abgleich von Bildschärfe und Vergrößerung ist eine Vorrichtung 16 bei der Adapteroptik 5 angeordnet, welche die Justage der einzelnen Linsen 11 bis 14 erlaubt. Figur 3 beschreibt unter anderem die Adapteroptik 5 näher.

Die nötigen Strahlumlenkungen werden einerseits durch das objektseitige Prisma 2, welches das durch die Okularoptik korrigierte reelle Zwischenbild 23 auf die Adapteroptik 5 umlenkt, realisiert. Andererseits lenkt das bildseitige Prisma 3 die Strahlen der Adapteroptik 5 auf die Sensoroberfläche 20 (d.h. einen Bildsensor), wobei der Bildsensor 20 parallel zur optischen Achse angeordnet ist. Zusammen mit der exzentrischen Anordnung der Adapteroptik 5 wird somit ein besonders kompakter Aufbau des Kameraobjektivs 1 realisiert. Ferner ist zum Schutz des Bildsensors 20 zwischen dem bildseitigen Prisma 3 und dem Bildsensor 20 eine planparallele Glasscheibe 15 angeordnet.

In Figur 2 ist eine mögliche Ausführung der auf der optischen Achse O1 angeordneten Okularoptik 4 schematisch dargestellt. Wie in Figur 2 gezeigt, umfasst die Okularoptik 4 eine bikonkave Linse 6, eine bikonvexe Linse 7, eine Stablinse 8, eine plan-konkave Linse 9 und eine bikonvexe Linse 10. Ferner zeigt Figur 2 das Zwischenbild 23. Die Linsen 6 bis 10 sind in der gezeigten Ausgestaltung in zwei Linsengruppen angeordnet. Eine erste Linsengruppe wird gebildet durch die bikonkave Linse 6 und die bikonvexe Linse 7. Die beiden Linsen 6, 7 der ersten Linsengruppe sind miteinander verkittet und bilden einen Achromaten. Ferner umfasst die Okularoptik 4 eine zweite Linsengruppe, bestehend aus der Stablinse 8, der plankonkaven Linse 9 und der bikonvexen Linse 10. Die Linsen 8, 9 und 10 der zweiten Linsengruppe sind miteinander verkittet. Die plan-konkave Linse 9 und die bikonvexe Linse 10 sind derart ausgebildet, dass sie einen Achromaten bilden. Die Stablinse 8 ist derart ausgebildet, dass sie die Bildfeldwölbung einer vorhergehenden Optik, beispielsweise des nicht gezeigten optischen Relaissystems, minimiert. Ferner erlaubt die Stablinse 8 eine Anpassung der Länge des Endoskops, ohne dabei den Schaftdurchmesser zu erhöhen. Zwischen der ersten und der zweiten Linsengruppe kann ein Luftspalt angeordnet sein.

Zur Verminderung von Streulicht weisen Oberflächen, die senkrecht zur optischen Achse O1 stehen und nicht mit einer anderen Oberfläche in Kontakt stehen, eine Antireflexionsbeschichtung 25 auf (siehe Figur 2).

Figur 3 zeigt einen bildseitigen Ausschnitt einer schematischen Darstellung des Kameraobjektivs 1. Dieser Ausschnitt umfasst die exzentrisch angeordnete Adapteroptik 5, den Bildsensor 20 und die Prismen 2, 3 zur Strahlumlenkung.

Das objektseitige Prisma 2 realisiert eine Strahlumlenkung von der optischen Achse O1 auf die optische Achse 02 der Adapteroptik 5. Das bildseitige Prisma 3 realisiert eine Strahlumlenkung von der optischen Achse 02 der Adapteroptik 5 auf den parallel zur optischen Achse O1 angeordneten Bildsensor 20. Die beiden Prismen 2, 3 weisen zur Vermeidung von Lichtverlust durch Transmission an den Seiten eine Hochreflexbeschichtung 24 auf, an denen die Strahlumlenkung erfolgt. Zur Vermeidung von Streulicht weisen weitere Seiten entweder eine Antireflexbeschichtung 25 oder eine in Figur 3 nicht gezeigte matt schwarze Beschichtung 26 auf (siehe Figur 4).

Die parallel zur optischen Achse O1 angeordnete Adapteroptik 5 umfasst eine konvex-plane Linse 11, eine Meniskuslinse 12, eine bikonvexe Linse 13 und eine bikonkave Linse 14. Die bikonvexe Linse 13 und die bikonkave Linse 14 sind miteinander verkittet und chromatisch korrigiert. Ferner ist die Vorrichtung 16 zum Verstellen der Adapteroptik 5 gezeigt. Die Vorrichtung 16 ist besonders vorteilhaft in einem stereoskopischen Endoskop, denn damit können die Bildschärfe und Vergrößerung für jeden der beiden Bildkanäle separat eingestellt werden.

Figur 4 zeigt einen bildseitigen Ausschnitt einer schematischen Darstellung des Kameraobjektivs 1. Im Unterschied zu Figur 3 ist die Darstellungsebene in Figur 4 parallel zum Bildsensor 20. Dieser Ausschnitt umfasst die exzentrisch angeordnete Adapteroptik 5 und die Prismen 2, 3 zur Strahlumlenkung. Insbesondere dargestellt ist die matt schwarze Beschichtung 26 der beiden Prismen 2, 3, welche zur Reduzierung von Streulicht dienen.

Tabelle 1 zeigt die Linsendaten des Kameraobjektivs 1 nach Figur 1 bis 4. Die optisch wirksamen Flächen sind in Tabelle 1 von der Objektseite her mit 1 bis 22 durchnummeriert. Alle Längenangaben sind in der Einheit [mm]. Die Bezeichnung der Gläser folgt der Nomenklatur von Schott.

**Tabelle 1**

| Fläche | Radius | Dicke | Glas | Durchmesser |
|---|---|---|---|---|
| Objekt | Unendlich | 0 | | 2,5 |
| 1 | Unendlich | 2,59 | | 2,5 |
| 2 | -9,12 | 0,5 | N-SF4 | 3,4 |
| 3 | 4,678 | 1 | N-LAF21 | 3,4 |
| 4 | -6,1 | 1,234 | | 3,4 |
| 5 | Unendlich | 14 | N-LAF21 | 3,4 |
| 6 | Unendlich | 0 | | 3,4 |
| 7 | Unendlich | 0,6999951 | N-SF15 | 3,2 |
| 8 | 8,64 | 1,45 | N-LAF21 | 3,2 |
| 9 | -14 | 4,1 | | 3,2 |
| 10 | Unendlich | 3 | | 3,6 |
| 11 | Unendlich | 19 | N-BK7 | 2,58688 |
| 12 | Unendlich | 3 | | 5,451527 |
| 13 | 22,16 | 1 | N-BK7 | 7 |
| 14 | Unendlich | 0,4999999 | | 7 |
| 15 | 15,125 | 1 | N-SK5 | 7 |
| 16 | 50 | 1,533036 | | 7 |
| 17 | 10,58 | 1,4 | N-LAK21 | 7 |
| 18 | -21,76 | 1 | LF5 | 7 |
| 19 | 6,2 | 11,9043 | | 5,8 |
| 20 | Unendlich | 7 | BK7 | 5,747444 |
| 21 | Unendlich | 0,5 | | 5,942784 |
| 22 | Unendlich | 0,5 | BK7 | 5,963899 |
| Aperturstop | Unendlich | 0,99 | | 5,977852 |
| Bild | Unendlich | - | | 6,058665 |

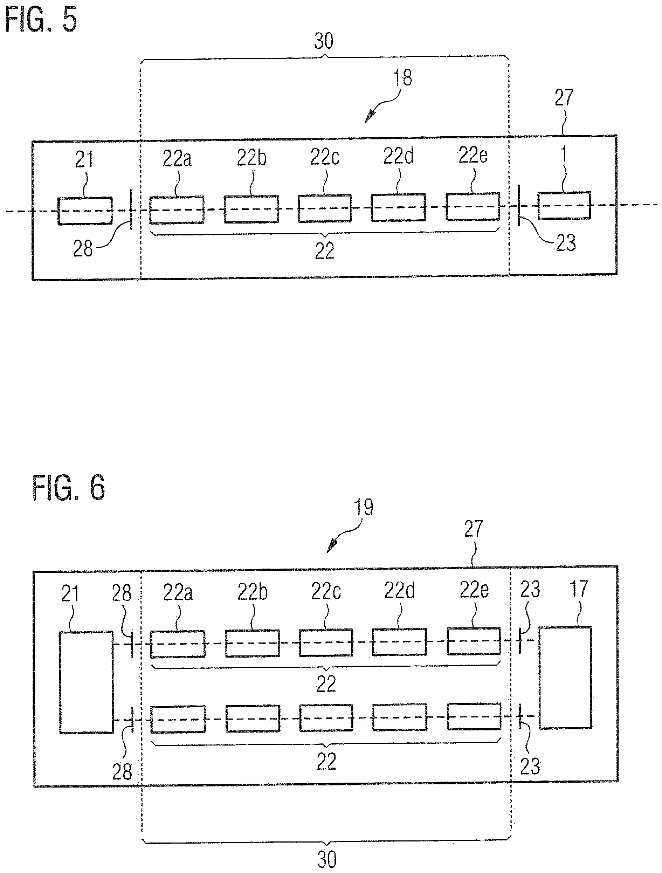

In Figur 5 ist ein Ausführungsbeispiel eines monokularen Endoskops 18 gezeigt, welches das Kameraobjektiv 1 nach Figur 1 bis 4 enthält. Das monokulare Endoskop 18 umfasst von der Objektseite her gesehen ein Objektiv 21, ein optisches Relaissystem 30 mit einem Relaismodul 22 mit mehreren Relaismodulkomponenten 22a bis 22e und das Kameraobjektiv 1. Ferner weist das Endoskop 18 einen Schaft 27 auf, in dem die vorgenannten Elemente angeordnet sind.

Das am distalen Ende des Endoskops 18 angeordnete Objektiv 21 erzeugt ein erstes Zwischenbild 28 des zu beobachtenden Objektes. Das Relaissystem 22 bildet das distale, erste Zwischenbild 28 auf ein proximales, zweites Zwischenbild 23 ab. Damit überträgt das Relaissystem 22 das erste Zwischenbild 28 gleichsam vom distalen zum proximalen Ende des Endoskops 18. Das am proximalen Ende des Endoskops 18 angeordnete Kameraobjektiv 1 bildet schließlich das zweite Zwischenbild 23 auf die in Figur 5 nicht gezeigte Sensoroberfläche 20 ab.

Ein Ausführungsbeispiel eines stereoskopischen Endoskops 19 ist in Figur 6 schematisch dargestellt. Gegenüber dem in Figur 5 dargestellten monokularen Endoskop 18 weist das stereoskopische Endoskop 19 zwei optische Kanäle auf. Das stereoskopische Endoskop 19 hat einen Schaft 27, in dem vom distalen Ende her betrachtet ein Objektiv 21, ein Relaissystem 30 mit zwei Relaismodulen 22 (stereoskopisches Relaissystem) und ein proximal angeordnetes Kameraobjektiv 17 angeordnet sind.

Das Objektiv 21 bildet das zu beobachtende Objekt auf zwei distale Zwischenbilder 28 ab, die jeweils einem optischen Kanal zugeordnet sind. Das stereoskopische Relaissystem 22 nach Figur 6 bildet je eines der beiden distalen Zwischenbilder 28 auf jeweils eines der beiden proximalen Zwischenbilder 23 ab. Das Kameraobjektiv 17 nach Figur 6 ist durch zwei Kameraobjektive 1 nach Figur 1 bis 4 gebildet. Jeweils eines der beiden Kameraobjektive 1 ist dabei einem der optischen Kanäle zugeordnet. Jedes der beiden Kameraobjektive 1 bildet schließlich je das proximale, zweite Zwischenbild 23 auf die entsprechende Sensoroberfläche 20 ab.

Bezeichnungsliste
- 1: Kameraobjektiv
- 2: objektseitiges Prisma
- 3: bildseitiges Prisma
- 4: Okularoptik
- 5: Adapteroptik
- 6: bikonkave erste Linse
- 7: bikonvexe zweite Linse
- 8: Stablinse (dritte Linse)
- 9: plan-konkave vierte Linse
- 10: bikonvexe fünfte Linse
- 11: konvex-plane sechste Linse
- 12: Meniskuslinse (siebente Linse)
- 13: bikonvexe achte Linse
- 14: bikonkave neunte Linse
- 15: plan-parallele Glasplatte
- 16: Justiervorrichtung
- 17: Stereoskopisches Kameraobjektiv
- 18: Monokulares Endoskop
- 19: Stereoskopisches Endoskop
- 20: Sensoroberfläche
- 21: Objektiv
- 22: Relaismodul
- 23, 28: Zwischenbild
- 24: HR-Beschichtung
- 25: AR-Beschichtung
- 26: Matt schwarze Beschichtung

- 27: Schaft
- 30: Relaissystem
- O1, O2: optische Achse

## Patentansprüche

1. Kameraobjektiv (1) für ein Endoskop (18, 19),
mit einer ersten optischen Achse (O1) und einer zweiten optischen Achse (02), die relativ zur ersten optischen Achse (O1) versetzt ist,
mit einem objektseitigen ersten Prisma (2) und einem bildseitigen zweiten Prisma (3),
mit einem objektseitig des ersten Prismas (2) angeordneten ersten Linsensystem (4) und einem bildseitig des ersten Prismas (2) angeordneten zweiten Linsensystem (5), wobei das erste Linsensystem (4) längs der ersten optischen Achse (O1) angeordnet ist, und wobei das zweite Linsensystem (5) längs der zweiten optischen Achse (02) angeordnet ist, und
mit einer am bildseitigen Ende des Kameraobjektivs (1) parallel zur ersten optischen Achse (O1) angeordneten Sensoroberfläche (20),
wobei das erste Linsensystem (4) eine Okularoptik und das zweite Linsensystem (5) eine Adapteroptik bildet, wobei das erste Prisma (2) und das zweite Prisma (3) dazu ausgebildet sind, eine erste bis dritte Strahlumlenkung als eine dreifache Strahlumlenkung zu bewirken, so dass mit Hilfe des ersten Prismas (2) die erste und zweite Strahlumlenkung als eine zweifache Strahlumlenkung von der ersten optischen Achse (O1) auf die zweite optischen Achse (02) und mit Hilfe des zweiten Prismas (3) die dritte Strahlablenkung als eine einfache Strahlumlenkung von der zweiten optischen Achse (02) auf die parallel zur ersten optischen Achse (O1) angeordnete Sensoroberfläche (20) bewirkt wird, **dadurch gekennzeichnet, dass** das erste Linsensystem (4) eine bikonkave erste Linse (6), eine bikonvexe zweite Linse (7), eine als Stablinse ausgebildete dritte Linse (8), eine plan-konkave vierte Linse (9) und eine bikonvexe fünfte Linse (10) in dieser Reihenfolge von der Objektseite her gesehen enthält,
und dass das zweite Linsensystem (5) eine konvex-plane sechste Linse (11), eine als Meniskuslinse ausgebildete siebente Linse (12), eine bikonvexe achte Linse (13) und eine bikonkave neunte Linse (14) in dieser Reihenfolge von der Objektseite her gesehen enthält.

2. Kameraobjektiv (1) nach Anspruch 1, **dadurch gekennzeichnet, dass** die erste optische Achse (O1) und die zweite optische Achse (02) parallel zueinander sind.

3. Kameraobjektiv (1) nach Anspruch 1 oder 2, **dadurch gekennzeichnet, dass** die bikonkave erste Linse (6) und die bikonvexe zweite Linse (7) miteinander verkittet sind.

4. Kameraobjektiv (1) nach einem der vorhergehenden Ansprüche, **dadurch gekennzeichnet, dass** die dritte Linse (8), die plan-konkave vierte Linse (9) und die bikonvexe fünfte Linse (10) miteinander verkittet sind.

5. Kameraobjektiv (1) nach einem der vorhergehenden Ansprüche, **dadurch gekennzeichnet, dass** die bikonvexe achte Linse (13) und die bikonkave neunte Linse (14) miteinander verkittet sind.

6. Kameraobjektiv (1) nach einem der vorhergehenden Ansprüche, **dadurch gekennzeichnet, dass** zwischen dem zweiten Prisma (3) und der Sensoroberfläche (20) eine planparallele Glasplatte (15) parallel zur Sensoroberfläche (20) angeordnet ist.

7. Kameraobjektiv (1) nach einem der vorhergehenden Ansprüche, **dadurch gekennzeichnet, dass** die bikonkave erste Linse (6), die bikonvexe zweite Linse (7), die als Stablinse ausgebildete dritte Linse (8), die plan-konkave vierte Linse (9) und/oder die bikonvexe fünfte Linse (10) des ersten Linsensystems (4) und/oder die konvex-plane sechste Linse (11), die als Meniskuslinse ausgebildete siebente Linse (12), die bikonvexe achte Linse (13) und/oder die bikonkave neunte Linse (14) des zweiten Linsensystems (5) an ihren frei gegen Luft liegenden Außenflächen jeweils eine Antireflexionsschicht (25) aufweisen.

8. Kameraobjektiv (1) nach einem der vorhergehenden Ansprüche, **dadurch gekennzeichnet, dass** die erste und zweite Strahlumlenkung an einer ersten Oberfläche (F1) beziehungsweise zweiten Oberfläche (F2) des ersten Prismas (2) und die dritte Strahlumlenkung an einer dritten Oberfläche (F3) des zweiten Prismas (3) erfolgt, und dass die erste und/oder zweite Oberfläche (F1, F2) des ersten Prismas (2) und/oder die dritte Oberfläche (F3) des zweiten Prismas (3) eine Hochreflexionsschicht (24) aufweisen.

9. Kameraobjektiv (1) nach einem der vorhergehenden Ansprüche, **dadurch gekennzeichnet, dass** eine oder mehrere senkrecht zum Strahlengang des Kameraobjektivs (1) stehenden Oberflächen des ersten Prismas (2) und/oder des zweiten Prismas (3) eine Antireflexionsschicht (25) aufweisen.

10. Kameraobjektiv (1) nach einem der vorhergehenden Ansprüche, **dadurch gekennzeichnet, dass** eine oder mehrere parallel zur ersten optischen Achse (O1) und senkrecht zur Sensoroberfläche (20) stehende Oberflächen des ersten Prismas (2) und/oder des zweiten Prismas (3) eine matt schwarze Beschichtung (26) aufweisen.

11. Kameraobjektiv (1) nach einem der vorhergehenden Ansprüche, **dadurch gekennzeichnet, dass** die bikonkave erste Linse (6), die bikonvexe zweite Linse (7), die als Stablinse ausgebildete dritte Linse (8), die plan-konkave vierte Linse (9) und/oder die bikonvexe fünfte Linse (10) des ersten Linsensystems (4) und/oder die bikonkave neunte Linse (14) des zweiten Linsensystems (5) aus einem Flintglas bestehen.

12. Kameraobjektiv (1) nach einem der vorhergehenden Ansprüche, **dadurch gekennzeichnet, dass** die konvex-plane sechste Linse (11), die als Meniskuslinse ausgebildete siebente Linse (12) und/oder die bikonvexe achte Linse (13) des zweiten Linsensystems (5) und/oder das erste Prisma (2) und/oder das zweite Prisma aus einem Kronglas bestehen.

13. Kameraobjektiv (1) nach einem der vorhergehenden Ansprüche, **dadurch gekennzeichnet, dass** das Kameraobjektiv (1) eine das zweite Linsensystem (5) umfassende Vorrichtung (16) zur Justierung der Bildschärfe und Vergrößerung aufweist.

14. Stereoskopisches Kameraobjektiv (17), umfassend zwei paarweise angeordnete Kameraobjektive (1) nach einem der vorhergehenden Ansprüche.

15. Monokulares Endoskop (18) oder stereoskopisches Endoskop (19), **dadurch gekennzeichnet, dass** das monokulare Endoskop (18) ein Kameraobjektiv (1) nach einem der Ansprüche 1 bis 13 umfasst, und dass das stereoskopische Endoskop (19) das stereoskopische Kameraobjektiv (17) nach Anspruch 14 umfasst.

## Claims

1. A camera objective lens (1) for an endoscope (18, 19), comprising a first optical axis (O1) and a second optical axis (02) which is offset relative to the first optical axis (O1),
an object-side first prism (2) and an image-side second prism (3),
a first lens system (4) arranged on the object side of the first prism (2) and a second lens system (5) arranged on the image side of the first prism (2), wherein the first lens system (4) is arranged along the first optical axis (O1), and wherein the second lens system (5) is arranged along the second optical axis (02), and
a sensor surface (20) arranged at the image-side end of the camera objective lens (1) parallel to the first optical axis (O1),
wherein the first lens system (4) forms an eyepiece optical system and the second lens system (5) forms an adapter optical system,
wherein the first prism (2) and the second prism (3) are configured to cause a first to third beam deflection as a three-fold beam deflection so that by means of the first prism (2) the first and second beam deflection is caused as a two-fold beam deflection from the first optical axis (O1) to the second optical axis (02) and by means of the second prism (3) the third beam deflection is caused as a simple beam deflection from the second optical axis (02) to the sensor surface (20) arranged parallel to the first optical axis (O1),
**characterized in that** the first lens system (4) includes a biconcave first lens (6), a biconvex second lens (7), a third lens (8) formed as a rod lens, a plane-concave fourth lens (9) and a biconvex fifth lens (10) in this order as viewed from the object side,
and that the second lens system (5) includes a convex-plane sixth lens (11), a seventh lens (12) formed as a meniscus lens, a biconvex eighth lens (13) and a biconcave ninth lens (14) in this order as viewed from the object side.

2. The camera objective lens (1) according to claim 1, **characterized in that** the first optical axis (O1) and the second optical axis (02) are parallel to each other.

3. The camera objective lens (1) according to claim 1 or 2, **characterized in that** the biconcave first lens (6) and the biconvex second lens (7) are cemented to each other.

4. The camera objective lens (1) according to one of the preceding claims, **characterized in that** the third lens (8), the plane-concave fourth lens (9) and the biconvex fifth lens (10) are cemented to each other.

5. The camera objective lens (1) according to one of the preceding claims, **characterized in that** the biconvex eighth lens (13) and the biconcave ninth lens (14) are cemented to each other.

6. The camera objective lens (1) according to one of the preceding claims, **characterized in that** a plane-parallel glass plate (15) is arranged parallel to the sensor surface (20) between the second prism (3) and the sensor surface (20).

7. The camera objective lens (1) according to one of the preceding claims, **characterized in that** the biconcave first lens (6), the biconvex second lens (7), the third lens (8) formed as a rod lens, the plane-concave fourth lens (9) and/or the biconvex fifth lens (10) of the first lens system (4) and/or the convex-plane sixth lens (11), the seventh lens (12) formed as a meniscus lens, the biconvex eighth lens (13) and/or the biconcave ninth lens (14) of the second lens system (5) each have an antireflection coating (25) at their outer surfaces that are exposed to air.

8. The camera objective lens (1) according to one of the preceding claims, **characterized in that** the first and second beam deflection occurs at a first surface (F1) and a second surface (F2) of the first prism (2), respectively, and the third beam deflection occurs at a third surface (F3) of the second prism (3), and that the first and/or second surface (F1, F2) of the first prism (2) and/or the third surface (F3) of the second prism (3) have a high-reflection coating (24).

9. The camera objective lens (1) according to one of the preceding claims, **characterized in that** one or more surfaces of the first prism (2) and/or of the second prism (3) that are perpendicular to the beam path of the camera objective lens (1) have an antireflection coating (25).

10. The camera objective lens (1) according to one of the preceding claims, **characterized in that** one or more surfaces of the first prism (2) and/or of the second prism (3) that are perpendicular to the sensor surface (20) and parallel to the first optical axis (O1) have a matt black coating (26).

11. The camera objective lens (1) according to one of the preceding claims, **characterized in that** the biconcave first lens (6), the biconvex second lens (7), the third lens (8) formed as a rod lens, the plane-concave fourth lens (9) and/or the biconvex fifth lens (10) of the first lens system (4) and/or the biconcave ninth lens (14) of the second lens system (5) are made of flint glass.

12. The camera objective lens (1) according to one of the preceding claims, **characterized in that** the convex-plane sixth lens (11), the seventh lens (12) formed as a meniscus lens and/or the biconvex eighth lens (13) of the second lens system (5) and/or the first prism (2) and/or the second prism are made of crown glass.

13. The camera objective lens (1) according to one of the preceding claims, **characterized in that** the camera objective lens (1) has a device (16), comprising the second lens system (5), for adjusting image sharpness and magnification.

14. A stereoscopic camera objective lens (17), comprising two camera objective lenses (1) according to one of the preceding claims arranged in pairs.

15. A monocular endoscope (18) or a stereoscopic endoscope (19), **characterized in that** the monocular endoscope (18) comprises a camera objective lens (1) according to one of the claims 1 to 13, and that the stereoscopic endoscope (19) comprises the stereoscopic camera objective lens (17) according to claim 14.

## Revendications

1. Objectif de caméra (1) destiné à un endoscope (18, 19),
avec un premier axe optique (O1) et un second axe optique (O2) décalé par rapport au premier axe optique (O1),
avec un premier prisme (2) côté objet et un second prisme (3) côté image,
avec un premier système de lentilles (4) agencé du côté objet du premier prisme (2) et un second système de lentilles (5) agencé du côté image du premier prisme (2), dans lequel le premier système de lentilles (4) est agencé le long du premier axe optique (O1), et dans lequel le second système de lentilles (5) est agencé le long du second axe optique (O2), et
avec une surface de capteur (20) agencée de manière parallèle au premier axe optique (O1) au niveau de l'extrémité côté image de l'objectif de caméra (1),
dans lequel le premier système de lentilles (4) forme une optique d'oculaire et le second système de lentilles (5) forme une optique adaptative,
dans lequel le premier prisme (2) et le second prisme (3) sont conçus pour provoquer des première à troisième déviations de faisceau sous la forme d'une triple déviation de faisceau de sorte que les première et deuxième déviations de faisceau sont provoquées à l'aide du premier prisme (2) sous la forme d'une double déviation de faisceau du premier axe optique (O1) vers le second axe optique (O2) et la troisième déviation de faisceau est provoquée à l'aide du second prisme (3) sous la forme d'une déviation unique de faisceau du second axe optique (O2) vers la surface de capteur (20) agencée de manière parallèle au premier axe optique (O1),
**caractérisé en ce que** le premier système de lentilles (4) contient une première lentille (6) biconcave, une deuxième lentille (7) biconvexe, une troisième lentille (8) réalisée sous forme de lentille barreau, une quatrième lentille (9) plan-concave et une cinquième lentille (10) biconvexe, considérées dans ledit ordre à partir du côté objet,
et **en ce que** le second système de lentilles (5) contient une sixième lentille (11) convexe-plan, une septième lentille (12) réalisée sous forme de lentille ménisque, une huitième lentille (13) biconvexe et une neuvième lentille (14) biconcave, considérées dans ledit ordre à partir du côté objet.

2. Objectif de caméra (1) selon la revendication 1, **caractérisé en ce que** le premier axe optique (O1) et le second axe optique (O2) sont parallèles l'un par rapport à l'autre.

3. Objectif de caméra (1) selon la revendication 1 ou 2, **caractérisé en ce que** la première lentille (6) biconcave et la deuxième lentille (7) biconvexe sont collées l'une à l'autre.

4. Objectif de caméra (1) selon l'une quelconque des revendications précédentes, **caractérisé en ce que** la troisième lentille (8), la quatrième lentille (9) plan-concave et la cinquième lentille (10) biconvexe sont collées les unes aux autres.

5. Objectif de caméra (1) selon l'une quelconque des revendications précédentes, **caractérisé en ce que** la huitième lentille (13) biconvexe et la neuvième lentille (14) biconcave sont collées l'une à l'autre.

6. Objectif de caméra (1) selon l'une quelconque des revendications précédentes, **caractérisé en ce qu'**une lame de verre (15) à faces parallèles est agencée entre le second prisme (3) et la surface de capteur (20) de manière parallèle à la surface de détection (20).

7. Objectif de caméra (1) selon l'une quelconque des revendications précédentes, **caractérisé en ce que** la première lentille (6) biconcave, la deuxième lentille (7) biconvexe, la troisième lentille (8) réalisée sous forme de lentille barreau, la quatrième lentille (9) plan-concave et/ou la cinquième lentille (10) biconvexe du premier système de lentilles (4) et/ou la sixième lentille (11) convexe-plan, la septième lentille (12) réalisée sous forme de lentille ménisque, la huitième lentille (13) biconvexe et/ou la neuvième lentille (14) biconcave du second système de lentilles (5) présentent respectivement une couche antireflet (25) au niveau de leurs surfaces extérieures exposées à l'air.

8. Objectif de caméra (1) selon l'une quelconque des revendications précédentes, **caractérisé en ce que** les première et deuxième déviations de faisceau ont lieu au niveau d'une première surface (F1) ou d'une deuxième surface (F2) du premier prisme (2) et la troisième déviation de faisceau a lieu au niveau d'une troisième surface (F3) du second prisme (3) a lieu, et **en ce que** la première et/ou la deuxième surface (F1, F2) du premier prisme (2) et/ou la troisième surface (F3) du second prisme (3) présente une couche à réflexion élevée (24).

9. Objectif de caméra (1) selon l'une quelconque des revendications précédentes, **caractérisé en ce qu'**une ou plusieurs surfaces du premier prisme (2) et/ou du second prisme (3) perpendiculaire (s) au trajet de faisceau de l'objectif de caméra (1) présente(nt) une couche antireflet (25).

10. Objectif de caméra (1) selon l'une quelconque des revendications précédentes, **caractérisé en ce qu'**une ou plusieurs surfaces du premier prisme (2) et/ou du second prisme (3) parallèle(s) au premier axe optique (O1) et perpendiculaire(s) à la surface de capteur (20) présente(nt) un revêtement noir mat (26).

11. Objectif de caméra (1) selon l'une quelconque des revendications précédentes, **caractérisé en ce que** la première lentille (6) biconcave, la deuxième lentille (7) biconvexe, la troisième lentille (8) réalisée sous forme de lentille barreau, la quatrième lentille (9) plan-concave et/ou la cinquième lentille (10) biconvexe du premier système de lentilles (4) et/ou la neuvième lentille (14) biconcave du second système de lentilles (5) sont constituées d'un verre flint.

12. Objectif de caméra (1) selon l'une quelconque des revendications précédentes, **caractérisé en ce que** la sixième lentille (11) convexe-plan, la septième lentille (12) réalisée sous forme de lentille ménisque et/ou la huitième lentille (13) biconvexe du second système de lentilles (5) et/ou le premier prisme (2) et/ou le second prisme sont constitués d'un verre crown.

13. Objectif de caméra (1) selon l'une quelconque des revendications précédentes, **caractérisé en ce que** l'objectif de caméra (1) présente un dispositif (16) comprenant le second système de lentilles (5) et destiné à ajuster la netteté d'image et le grossissement.

14. Objectif de caméra stéréoscopique (17), comprenant deux objectifs de caméra (1), agencés par paires, selon l'une quelconque des revendications précédentes.

15. Endoscope monoculaire (18) ou endoscope stéréoscopique (19), **caractérisé en ce que** l'endoscope monoculaire (18) comprend un objectif de caméra (1) selon l'une quelconque des revendications 1 à 13, et **en ce que** l'endoscope stéréoscopique (19) comprend l'objectif de caméra stéréoscopique (17) selon la revendication 14.
